# EUROPEAN PATENT APPLICATION

(11) **EP 2 426 205 A1**
(43) Date of publication of application: **07.03.2012**
(21) Application number: 10769367.3
(22) Date of filing: 26.04.2010
(51) Int. Cl.: C12N 15/82, A01H 5/00

(54) **GENES HOMOLOGOUS TO THE FLOWERING LOCUS T (FT) GENE AND THE USE THEREOF FOR MODULATING TUBERIZATION**

(30) Priority: 29.04.2009 ES 200930114
(71) Applicant: Consejo Superior de Investigaciones Cientificas (CSIC), 28006 Madrid (ES); HZPC Holland B.V. R&D Manager (20%), 9123 Metslawier (NL)
(72) Inventor: PRAT, Salomé, E-28049 Madrid (ES); NAVARRO, Cristina, E-28049 Madrid (ES); ABELENDA, José A., E-28049 Madrid (ES); VAN DOORN, J. E., NL-9123 Metslawier (NL)
(74) Representative: Pons Ariño, Angel
(86) International application number: PCT/ES2010/070265
(87) International publication number: WO 2010/125221

(57) **Abstract**

Use of genes homologous to the FT gene of Arabidopsis thaliana of any plant species for modulating tuberization in plants that develop tubers, nucleotide sequence that encodes potato SP6A protein (homologous to FT of Arabidopsis thaliana), gene constructs that express said gene and the use thereof.

## Description

The present invention falls within the fields of biology, molecular biology and biotechnology, and refers to the nucleotide sequence that encodes the potato SP6A protein and the use thereof, genetic constructs that express the potato SP6A gene, as well as the use of genes homologous to the FT gene of *Arabidopsis thaliana* of any vegetable species, for modulating tuberization in potato plants or other species that develop tubercles. As a proof of concept, the SP6A gene has been expressed under the regulation of a heat-shock promoter corresponding to the soybean *hs6871* gene. The expression of the SP6A/FT gene at temperatures greater than 30ºC acts as a tuberization induction signal; for this reason, the potato plants that integrate said constructs show a high yield of tubercles in geographical areas with high night-time temperatures.

### STATE OF THE ART

Potatoes (*Solanum tuberosum* L.) are one of the four most important crops worldwide, jointly with wheat, rice and corn, with the advantage that their tubercles have a greater yield per hectare than cereal grains. Tubercles or potatoes are used in animal feeding and for human consumption, directly cooked, in various processed foods, as a gelling agent and in the production of alcoholic beverages. Moreover, the products derived therefrom have numerous industrial applications, such as, for example, potato starch, which provides a coating for paper and textile products. The potato plant is an annual, herbaceous, dicotyledonous plant that propagates vegetatively, and in certain environments may also grow as a perennial plant. In general, it propagates from the tubercles (seed tubers), although it may also multiply from the seeds, called TPS (true potato seeds).

Tuberization is an important plant survival mechanism (van den Berg et al., 1996. Theor Appl Genet 93: 307-316), which involves environmental factors as well as genetic factors. Formation of the tubercle is preceded by initiation and growth of an underground shoot (the stolon), which, depending on the environmental conditions, stops growing longitudinally in order to initiate the formation of a storage organ (the tubercle). This process is associated with changes at both the morphological and biochemical levels. At the morphological level, a thickening of the sub-terminal area of the stolon is observed; said thickening is characterised, at the cellular level, by an increase in the size of the cells and an activation of cell division (Vreugdenhil et al., 1999. Ann Bot 84: 675-680) that is accompanied by a change in the division plane (Xu et al., 1998. J Exp Bot 49: 573-582). The biochemical processes include a change in the sucrose transport mechanism, from apoplastic to symplastic (Viola et al., 2001. Plant Cell 13: 385-398), starch synthesis (Tauberger et al., 2000. Plant J 23: 43-53) and the accumulation of reserve proteins (Taylor et al., 1992. Plant Mol Biol 20: 641-651).

The differentiation of tubercles takes place in response to certain given environmental conditions. Short days, low temperatures and fertilisers with a low nitrogen content promote tuberization, whereas the latter is delayed or inhibited by long days, high temperatures and nitrogen-rich fertilisers (Rodriguez-Falcon et al., Annu Rev Plant Biol 57: 151-180). The degree of dependency on these conditions varies with the genetic variety. Thus, the subspecies *andigena* requires short days in order to tuberize, whereas commercial varieties, primarily those derived from the subspecies *chilense,* are less dependent on these conditions and may form tubercles during long days. Regardless of this variable photoperiodic response, exposure to high temperatures (heat stress), particularly at night, strongly inhibits the production of tubercles in all potato varieties, thereby limiting the cultivation thereof to colder mountain areas, in geographical areas located close to the Equator.

This strong dependency on environmental conditions has made it necessary to select potato varieties with a better adaptation capacity to the various climates and temperature regimes characteristic of the different production areas. However, all these varieties still show optimal development in moderate climates (with daytime temperatures ranging between 20ºC-30ºC and night-time temperatures ranging between 8ºC-15ºC, combined with sufficient rain or irrigation water), when evaluated in terms of production and other relevant agronomical characteristics, such as average size, size distribution, tubercle shape and aesthetic aspects, such as depth of the eyes, etc. Climactic conditions wherein daytime temperatures are high (e.g. > 35ºC) and/or night-time temperatures exceed a minimum (e.g. > 20ºC) cause a dramatic decrease in the agricultural production of most potato cultivars and, therefore, are considered to be heat stress conditions. This makes it necessary to adequately select the planting date for the different varieties, on the basis of the climactic conditions of the cultivation area, in order to prevent the plant from being subjected to heat stress conditions at the time when the tubercles are formed. Thus, current varieties may be classified into varieties adapted to oceanic climates, Mediterranean climates, and continental or desertic climates, depending upon whether they show optimal development under the temperature, rainfall, irradiation and length-of-day conditions typical of these climates. Nevertheless, seasonal and climactic variations are often unpredictable factors that have a negative influence on the development of these varieties in the different commercial production areas. Therefore, the obtainment of varieties capable of tolerating different conditions or adapting to adverse heat stress conditions is an important pre-requisite that must be taken into consideration when selecting new varieties with a high production rate.

In recent decades, the cross-breeding of parental lines to improve their capacity to adapt to heat stress has made it possible to select a number of European commercial varieties with a high production and agronomical success in areas surrounding the Mediterranean Sea: Southern Europe, Middle East and Northern Africa, as well as Central Europe. Some examples of these varieties are Spunta, Sylvana, Mondial and Désirée. However, since potatoes are a very important food worldwide, with a greater yield per hectare than cereal grains, the cultivation thereof in developing countries, primarily agricultural countries, is of special interest. Consequently, there is a need to find new varieties that make it possible to improve the yield of this crop in regions with warm climates and long days.

It is well-known that daytime changes in temperature and length of day are perceived by the leaves. Under favourable conditions, leaves synthesise a mobile substance, or "tuberigen", which is transported to the underground stems, or stolons, to induce their differentiation into tubercles. During short days (SD) or at low temperatures, a greater amount of this substance is produced than during long days (LD), which causes the plant to produce more tubercles. Several transcription factors have been reported, such as POTM1 (Kang & Hannapel 1995. Gene 166: 329-330), POTH1 (Rosin et al., 2003. Plant Physiol 132: 106-117), StBEL5 (Chen et al., 2003. Plant J 38: 276-284) and an orthologue of the *CONSTANS* gene of *Arabidopsis thaliana* (Rodriguez-Falcon et al., 2006. Annu Rev Plant Biol 57: 151-180), that regulate the formation of tubercles in potatoes independently from or in combination with other factors.

In *Arabidopsis,* AtCO induces flowering under LD conditions and the overexpression thereof in potatoes causes a delay in tuberization under SD conditions (Martínez-García et al. 2002. Proc Natl Acad Sci USA 99: 15211-15216). The adverse effect of AtCO is exerted on the leaves, where it inhibits the production of the mobile tuberization signal, whose chemical or molecular identity was still unknown. Since there is indicative evidence that the signals that induce flowering and tuberization are similar, it was postulated that a conserved photoperiodic pathway, comprised by the *CONSTANS* and *FLOWERING LOCUS T (FT)* genes, could be involved in the induction of tuberization during SD (Rodriguez-Falcon et al., Annu Rev Plant Biol 57: 151-180). CO is a transcription factor of the zinc-finger type that is primarily expressed in the accompanying cells of the phloem, where it induces the expression of *FLOWERING LOCUS T (FT).* On the other hand, *FT* is a gene that shares homology with Raf-kinase type inhibitors and acts as a strong flowering inducer. In fact, it has recently been demonstrated that the FT protein moves through the phloem to the stem apical meristem, where it induces the transition from vegetative to inflorescence meristem, thereby initiating flowering.

### DESCRIPTION OF THE INVENTION

The authors of the present invention have identified and sequenced the potato gene orthologous to the *FLOWERING LOCUS T* (*FT*) gene of *Arabidopsis thaliana,* and have noticed that transgenic plants of the *andigena* potato variety that overexpress this gene may tuberise under long-day (LD) conditions. This shows that FT also acts as a tuberization induction signal. Since phasing of the endogenous circadian clock takes place both in response to changes in light/darkness and in response to fluctuations in temperature (high during the day but low during the night), the inhibition of tuberization at high temperatures may be the result of a phase change in the clock, which causes a lack of activation of the FT gene. In this invention, the authors have generated transgenic plants of the *Solanum tuberosum* L. cv *Spunta* variety that carry a construct consisting of the soybean heat-shock promoter (HSP), which is activated at temperatures greater than 35ºC, fused to the potato FT gene orthologue. It has been demonstrated that these plants present a greater production of tubercles at high temperatures (night-time temperatures of 25ºC) and, therefore, are resistant to heat stress.

Therefore, a first aspect of the invention relates to an isolated RNA or DNA polynucleotide, hereinafter called polynucleotide of the invention, capable of being translated into an amino acid sequence, hereinafter amino acid sequence of the invention, which comprises a peptide at least 85% identical to SEQ ID NO: 1. More preferably, the identity is 90%, 95% and, even more preferably, 99%.

SEQ ID NO: 1 comprises the amino acid sequence encoded by the *SP6A* gene of *Solanum tuberosum* L. ssp *andigena.* Said *SP6A* gene is a gene orthologous to the *FT* gene (*FLOWERING LOCUS T*) of *Arabidopsis thaliana. A* nucleotide sequence capable of being translated into SEQ ID NO: 1 could be, without being limited thereto, the sequence SEQ ID NO: 8.

The overall identity of the sequences homologous to the isolated sequence of the species *Solanum tuberosum* L. ssp *andigena* at the level of the amino acid sequence SEQ ID NO: 1 may be expected to be 80% or greater, and, more preferably, 85% or greater and, more preferably, 90%, 95% or 99%. The correspondence between the amino acid sequence of the putative sequence(s) and the sequence SEQ ID NO: 1 may be determined by methods known in the state of the art.

The term "isolated", as used in this specification, refers to nucleotides or peptides that: 1) are substantially free of components that normally accompany or interact with them in nature, or 2) if they are in their natural environment, have been synthetically (not naturally) altered by human intervention and/or introduced into a cell that does not have them in native form. For example, a natural nucleotide becomes "isolated" if it has been altered, or if it comes from a DNA that has been altered by human intervention (by means of, for example, without being limited thereto, directed mutagenesis, insertions, deletions, etc.). Likewise, a natural nucleotide becomes "isolated" if it is introduced into a genome that is not native to said nucleotide by non-natural means (transfection). Therefore, in this latter case, the term "isolated" is equivalent to the term "heterologue".

The term "homology", as used in this specification, refers to the similarity between two structures due to a common evolutionary ascendancy, or a common function, and, more specifically, to the similarity between two or more nucleotide or amino acid sequences. Since two sequences are considered to be homologous if they have the same evolutionary origin or if they have a similar function and structure, in general it is assumed that similarity or identity values greater than 60% indicate homology. Therefore, we may consider that identity percentages of, at least, 85% will preserve the function of the amino acid sequence comprised in SEQ ID NO: 1.

The term "orthologous" refers to homologous structures of different species, which have a common ancestor and, specifically, to the similarity between two or more nucleotide or amino acid sequences.

The term "identity", as used in this specification, refers to the proportion of identical nucleotides or amino acids in two nucleotide or amino acid sequences being compared. Sequence comparison methods are known in the state of the art, and include, without being limited thereto, the GAG programme, including GAP (Devereux et al., Nucleic Acids Research 12: 287 (1984) Genetics Computer Group University of Wisconsin, Madison, (WI)); BLAST, BLASTP or BLASTN, and FASTA (Altschul et al., J. Mol. Biol. 215: 403-410 (1999).

In a particular embodiment of the invention, the isolated RNA or DNA polynucleotide of the invention is capable of being translated into amino acid sequence SEQ ID NO: 1.

Another aspect of the invention provides a DNA or RNA genetic construct, hereinafter genetic construct of the invention, which comprises one of the following types of sequences:
a) nucleotide sequence, which comprises, at least, the polynucleotide of the invention, or the coding sequence of SEQ ID NO: 1, for the *in vitro* or *in vivo* transcription thereof, or
b) nucleotide sequence, corresponding to a gene expression system or vector, which comprises the polynucleotide of the invention, operatively linked to, at least, one promoter that directs the transcription of said nucleotide sequence, and to other necessary or appropriate sequences for the adequate transcription and regulation in time and space thereof, for example, initiation and termination signals, polyadenylation signal, transcriptional enhancers, etc.

Many of these expression constructs, systems or vectors may be obtained by conventional methods known to persons skilled in the art, and are a part of the present invention.

In another preferred embodiment, the promoter is an inducible promoter. Preferably, the promoter is capable of directing the transcription of a polynucleotide at temperatures greater than 17ºC, more preferably at temperatures greater than 20ºC, 25ºC, 30ºC, 35ºC, or, more preferably, at temperatures greater than 37ºC. In a preferred embodiment of this aspect of the invention, the promoter is preferably a heat-shock gene promoter (heat-shock promoter). In a more preferred embodiment, the promoter is the soybean heat-shock gene promoter (*hs6871*).

A "vector" is a replicon whereto another polynucleotide segment has been linked, in order to perform the replication and/or expression of the linked segment.

A "replicon" is any genetic element that behaves as an autonomous unit of polynucleotide replication inside a cell; i.e. capable of being expressed under its own control.

"Control sequence" refers to polynucleotide sequences that are necessary for the expression of the sequences whereto they are linked. The nature of said control sequences differs as a function of the host organism; in prokaryotes, said control sequences generally include a promoter, a ribosomal binding site and termination signals; in eukaryotes, said control sequences generally include promoters, termination signals, enhancers and, occasionally, silencers. The term "control sequences" is intended to include, at least, all the components the presence whereof is necessary for expression, and may also include additional components the presence whereof is advantageous.

"Operatively linked" refers to a juxtaposition wherein the components thus described have a relationship that allows them to function in the intended manner. A control sequence "operatively linked" to a sequence that is transcribed into the nucleotide sequence of the invention is linked in such a way that expression of the coding sequence is achieved under conditions compatible with the control sequences.

A "coding sequence" is a polynucleotide sequence that is transcribed into mRNA and translated into a polypeptide when it is under the control of appropriate regulatory sequences. The limits of the coding sequence are determined by means of a translation initiation codon at the 5'-end and a translation termination codon at the 3'-end. A coding sequence may include, without being limited thereto, mRNA, cDNA and recombinant polynucleotide sequences.

The terms "polynucleotide" and "nucleic acid" are used interchangeably herein, and refer to polymeric nucleotide forms of any length, both ribonucleotides (RNA) and deoxyribonucleotides (DNA).

The terms "amino acid sequence", "peptide", "oligopeptide", "polypeptide" and "protein" are used interchangeably herein, and refer to a polymeric amino acid form of any length, which may be coding or non-coding, chemically or biochemically modified.

Heat-shock genes are genes that encode heat-shock proteins, or HSPs (Tissieres et al., 1974. J Mol Biol 84: 389-398). Currently, it is widely accepted that all organisms have a common heat shock response mechanism, which involves big changes in gene expression patterns and the activation of HSPs. The importance of these proteins lies in their activity as molecular chaperones, which allow for survival of the cell under stress conditions (Hartl 1996. Nature 381: 571-579). The heat shock response is triggered when organisms are exposed to temperatures that exceed those considered to be normal for the correct growth thereof. Consequently, the temperature whereat this response is triggered varies as a function of each organism. Aside from temperature, there is a wide variety of stimuli capable of triggering this response, such as ultraviolet radiation, heavy metals or the lack of nutrients, amongst others (revised by Morimoto et al., 1992. J Biol Chem 267: 21987-21990). These observations have led to heat-shock proteins also being called stress proteins.

Within the context of the present invention, soybean (*Glycine max*) heat-shock gene promoter or promoter sequence is understood as a nucleotide or polynucleotide sequence that constitutes the regulatory sequence of the soybean heat-shock protein (hs6871), which would comprise various variants from:
a) nucleic acid molecules that comprise the nucleotide sequence SEQ ID NO:3,
b) nucleic acid molecules the complementary chain whereof hybridises with the polynucleotide sequence of a),
c) nucleic acid molecules that are at least 60%, 80%, 90%, 95%, 98% or 99% identical to SEQ ID NO: 3, wherein said nucleic acids have the activity and characteristics of the soybean heat-shock promoter.

The genetic construct of the invention could be used to express the amino acid sequence of the invention. Therefore, another aspect relates to the use of the genetic construct of the invention to express the amino acid sequence of the invention.

In this specification, FT (*FLOWERING LOCUS T*) is a gene that encodes a protein involved in the regulation of plant growth, by controlling the rate of floral transition. For example, it has been observed that an increase in the function of FT produces early flowering (Kardailsky et al., 1999. Science 286: 1962-1965). It has also been observed that it is responsible for the photoperiod response and, jointly with the CONSTANS (CO) gene, it is responsible for inducing flowering in response to variations in the length of day (photoperiod). It shares homology with Raf-kinase type inhibitors and acts as a strong flowering inducer.

Within the context of the present invention, FT (*FLOWERING LOCUS T*) is also defined by a nucleotide or polynucleotide sequence that constitutes the coding sequence of the FLOWERING LOCUS T protein, which would comprise different variants from:
a) nucleic acid molecules which encode a polypeptide that comprises the amino acid sequence of SEQ ID NO: 2,
b) nucleic acid molecules the complementary chain whereof hybridises with the polynucleotide sequence of a),
c) nucleic acid molecules the sequence whereof is different from a) and/or b) due to degeneration of the genetic code,
d) nucleic acid molecules which encode a polypeptide that comprises the amino acid sequence with an identity of at least 60%, 80%, 90%, 95%, 98% or 99% with respect to SEQ ID NO: 2, wherein the polypeptide encoded by said nucleic acids has the activity and structural characteristics of the FLOWERING LOCUS T protein.

A nucleotide sequence capable of being translated into SEQ ID NO: 2 could be, without being limited thereto, the sequence SEQ ID NO: 9.

In this invention, it has been observed that a polynucleotide orthologous to the *FT* gene of *Arabidopsis thaliana* (the SP6A gene of *S. tuberosum* ssp. *andigena*) is capable of inducing tuberization. Moreover, experiments have been performed with other homologues of the FT gene of *Arabidopsis thaliana* different from the SP6A gene.

Thus, the FT gene and other genes homologous to the *FT* (FLOWERING LOCUS T) gene of *Arabidopsis thaliana* would be capable of inducing tuberization.

Therefore, another aspect of the invention relates to the use of a polynucleotide homologous to the isolated FT gene (*FLOWERING LOCUS T*), in order to induce tuberization. In a preferred embodiment of this aspect of the invention, the polynucleotide homologous to the isolated FT (*FLOWERING LOCUS T*) gene is operatively linked to an inducible promoter and, preferably, to a heat-shock gene promoter. In an even more preferred embodiment of this aspect of the invention, the promoter is the soybean heat-shock promoter (hs6871). In another preferred embodiment of this aspect of the invention, the plant wherein tuberization is induced belongs to the genus *Solanum.* In another preferred embodiment, it belongs to the species *Solanum tuberosum* L. In a more preferred embodiment, the plant wherein tuberization is induced belongs to the *Spunta* cultivar of the species *Solanum tuberosum* L.

As polynucleotides homologous to the FT (*FLOWERING LOCUS T*) gene, we may also consider, without being limited thereto, any polynucleotide capable of being translated into biologically active variants or fragments of amino acid sequence SEQ ID NO: 2, where at least one amino acid has been selectively inserted, deleted or substituted, and which may induce tuberization in a plant. As regards the nucleotide sequences, a large number of these may lead to the same amino acid sequence due to degeneration of the genetic code. For example, the GCA, GCC, GCG and GCU codons all encode the amino acid alanine. Consequently, at each position where an alanine is encoded by a codon, the codon may be changed to any of the codons described without altering the peptide encoded. An average person skilled in the art knows that every nucleotide codon (except for AUG, which is commonly the only codon for methionine, and UGG, which is commonly the only codon for tryptophan) may be modified in order to obtain a functionally identical protein. As regards the amino acid sequences, an average person skilled in the art recognises that individual substitutions, additions or deletions of a nucleic acid, peptide, polypeptide or protein may alter, add or delete a single amino acid or a group of amino acids, conserving the biological activity of said peptide, or a similar activity (at least 30%, 40%, 50%, 60%, 70%, 80% or 90% of the original peptide), especially when an amino acid is substituted with a chemically similar amino acid. The following amino acids are chemically similar:
- Alanine (A), serine (S), threonine (T);
- Aspartic acid (D), glutamic acid (E);
- Asparagine (N), glutamine (Q);
- Arginine (R), Lysine (K);
- Isoleucine (I), Leucine (L), methionine (M), valine (V), and
- Phenylalanine (F), tyrosine (Y), tryptophan (W).

In a preferred embodiment of this aspect of the invention, the polynucleotide homologous to the FT (*FLOWERING LOCUS T*) gene is an isolated polynucleotide of the invention.

Another aspect of the invention relates to the use of the genetic construct of the invention to modulate tuberization in a plant.

In another preferred embodiment of this aspect of the invention, the plant wherein tuberization is induced belongs to the genus *Solanum.* In another preferred embodiment, it belongs to the species *Solanum tuberosum* L. In a more preferred embodiment, the plant wherein tuberization is induced belongs to the *Spunta* cultivar of the species *Solanum tuberosum* L.

In this specification, "plant" is understood to mean all those organisms that may be classified within the kingdom *Viridiplantae,* which includes green algae and terrestrial plants (*Embryophyta*).

The organisms of the genus *Solanum* belong to the Superkingdom *Eukaryota,* Kingdom *Viridiplantae,* Phylum *Streptophyta,* Subclass *Asterides,* Order *Solanales,* Family *Solanaceae.*

The organisms of the species *Solanum tuberosum* belong to the Superkingdom *Eukaryota,* Kingdom *Viridiplantae,* Phylum *Streptophyta,* Subclass *Asterides,* Order *Solanales,* Family *Solanaceae* and Genus *Solanum.*

The Spunta cultivar is a variety of *Solanum tuberosum,* registered in the Dutch National Registry of Varieties in 1968, the protection whereof (in the sense of the Community Plant Variety Office, CPVO) ended in 1998. It was developed by HZPC and is **characterised in that** it is a very productive early variety, well-adapted to different latitudes. This cultivar produces elongated, white-skin, yellow-flesh tubercles, which are suitable to be consumed fried, such as potato chips.

Another aspect relates to a seed, hereinafter called seed of the invention, the genetic material whereof integrates the polynucleotide of the invention, the genetic construct of the invention or a polynucleotide homologous to the FT (*FLOWERING LOCUS T*) gene. Preferably, the seed of the invention may be taxonomically classified as belonging to the genus *Solanum* and, more preferably, to the species *Solanum tuberosum* L.

Another aspect relates to a vegetable cell, hereinafter vegetable cell of the invention, the genetic material whereof integrates the polynucleotide of the invention, the genetic construct of the invention or a polynucleotide homologous to the FT (*FLOWERING LOCUS T*) gene. Preferably, the vegetable cells of the invention may be taxonomically classified as belonging to the genus *Solanum* and, more preferably, to the species *Solanum tuberosum* L.

Another aspect relates to a vegetable cell culture, hereinafter vegetable cell culture of the invention, the genetic material whereof integrates the polynucleotide of the invention, the genetic construct of the invention or a polynucleotide homologous to the FT (*FLOWERING LOCUS T*) gene. Preferably, the vegetable cells of the culture of the invention may be taxonomically classified as belonging to the genus *Solanum* and, more preferably, to the species *Solanum tuberosum* L.

In this specification, the term "cell culture" refers to a culture of cells isolated from the same or a different type of tissue, or to a collection of such cells organised into parts of a plant or into tissues (tissue cultures). Some types of cultures of this type are, for example, cultures of protoplasts, *calli* (group of *callus* or undifferentiated vegetable cells capable of regenerating a complete plant) and vegetable cells that are isolated from plants or parts of plants, such as, for example, without being limited thereto, embryos, protoplasts, meristematic cells, pollen, leaves or anthers.

Another aspect of the invention relates to a group of cells the genetic material whereof integrates the polynucleotide of the invention or the genetic construct of the invention, and which form the tubercles, the minitubercles or the microtubercles.

"Minitubercles", or "seed potatoes", are known to be small tubercles no greater than 3 cm in diameter used to create large commercial potato plantations. In the absence thereof, medium-size tubercles or pieces thereof that have at least one eye (i.e. a bud) are used.

Another aspect relates to a plant that comprises the cells or the vegetable cell culture of the invention. In a preferred embodiment of this aspect of the invention, the plant is obtained following the growth of a cell, a group of vegetable cells or the seed of the invention. Preferably, the plant may be taxonomically classified as belonging to the genus *Solanum* and, more preferably, to the species *Solanum tuberosum* L. Therefore, the genetic material of said plant will integrate the polynucleotides of the invention, the genetic constructs of the invention, or the FT gene or any of the homologues thereof, which are capable of inducing tuberization, regulated or not by a heat-shock promoter. In another preferred embodiment, the plant is capable of tuberising at temperatures greater than 17ºC, more preferably, at temperatures greater than 20ºC, 25ºC, 30ºC, 35ºC, or, more preferably, at temperatures greater than 37ºC.

Another aspect relates to a plant that is capable of tuberising at temperatures different from those whereat a control type plant of the same taxonomic category tuberises, due to non-transgenic mutations in the gene orthologous to the FT gene. Preferably, the plant may be taxonomically classified as belonging to the genus *Solanum* and, more preferably, to the species *Solanum tuberosum* L. In another preferred embodiment, the plant is capable of tuberising at temperatures greater than 17ºC, more preferably, at temperatures greater than 20ºC, 25ºC, 30ºC, 35ºC, or, more preferably, at temperatures greater than 37ºC.

Another aspect of the invention relates to a pollen grain the genetic material whereof is a derivative of the genetic material of a vegetable cell or group of cells of the invention, or which comes from a plant that is capable of tuberising at temperatures different from those whereat a control type plant of the same taxonomic category tuberises, due to non-transgenic mutations in the gene orthologous to the FT gene. Preferably, the pollen grain may be taxonomically classified as belonging to the genus *Solanum* and, more preferably, to the species *Solanum tuberosum* L.

Another aspect of the invention relates to the use of a polynucleotide homologous to the FT (*FLOWERING LOCUS T*) gene of *Arabidopsis thaliana,* operatively linked or not to an inducible promoter, preferably of a heat-shock gene and, more preferably, of the soybean heat-shock gene, which is integrated in the genetic material of the seed, cell, cell culture, plant or pollen grain of a plant of the invention, in order to induce tuberization.

Another aspect of the invention provides a method of obtaining plants capable of tuberising at temperatures different from those whereat a control type plant of the same taxonomic category tuberises, which comprises:
a) transfecting a polynucleotide homologous to the FT gene of *Arabidopsis thaliana,* the polynucleotide or the genetic construct of the invention in a host cell or vegetable cell culture,
b) growing the host cell or vegetable cell culture in a suitable medium, until a complete plant is regenerated.

In a preferred embodiment of this aspect of the invention, the cell, the vegetable cell culture and/or the plant may be taxonomically classified as belonging to the genus *Solanum* and, more preferably, to the species *Solanum tuberosum* L.

The method for modulating, preferably inducing, the tuberization process in a plant provided by the invention comprises any plant transformation process wherein the allogenous elements introduced comprise a polynucleotide homologous to the FT gene of *Arabidopsis thaliana,* the polynucleotide of the invention or the genetic construct of the invention.

In a particular embodiment of the invention, the "allogenous elements" comprise a soybean *HSP* gene promoter, the coding region corresponding to the potato *SP6A* gene and the CaMV 35S terminator, the kanamycin resistance gene (*nptll*) under the control of the *nos* promoter and the sequences comprised between the left border (LB) and the right border (RB) of the T-DNA of the pK7WGF2 binary vector (Karimi et al., 2002. Trends Plant Sci 7: 193-195).

The obtainment of transgenic plants is possible thanks to a characteristic of vegetables: totipotency, whereby any cell of a vegetable has the potential to regenerate a complete plant. The vegetable cells may be cultured under sterile conditions (in order to prevent pathogenic infections) in an artificial medium that provides the necessary nutrients for cell divisions and vegetative proliferation. There are three approaches to regenerate complete plants *in vitro*:
- Culturing of embryos: Isolation of zygotic embryos, promoting their growth as a plant in an artificial medium.
- Somatic or asexual embryogenesis: Generation of embryos from somatic tissues, such as microspores or leaves.
- Organogenesis: Generation of organs such as stems or roots from various tissues.

Since the genetic manipulation required introducing the transgenes acts at the cellular level, it is necessary to develop an adequate *in vitro* tissue culture technology for each vegetable species. In this way, the initially transformed cells will regenerate, by means of vegetative propagation, a complete plant where all the cells contain the transgene.

Basically, there are three techniques that make it possible to obtain transgenic plants:
- Protoplast transformation.
- Biolistic transformation (or bombardment with microprojectiles).
- Transformation by means of *Agrobacterium.*

Protoplast is the name given to vegetable cells without a cell wall. They are obtained by mechanical and enzymatic processes that eliminate the cell wall. This process makes it possible to obtain a suspension with millions of individual cells susceptible to being transformed. The protoplasts are kept in a culture medium and the gene to be transferred is added. In order to achieve penetration of the transgene, it is necessary to permeabilise the membrane, which is performed by different processes:
- Electroporation: It consists of applying electric shocks to the protoplast, such that the membrane is de-polarised and tiny pores are created wherethrough the DNA may penetrate.
- Treatment with polyethylene glycol in order to destabilise the cell membrane.
- Fusion of liposomes containing the DNA to be transferred with the membrane.

Once the DNA is integrated, it is necessary to culture the protoplasts in order to allow for the division thereof, under those conditions that make it possible for the plant that has integrated the transgene to regenerate.

Biolistics or bio-ballistics is the name given to the introduction of DNA into cells by means of the acceleration (shooting) of very small-size projectiles (microprojectiles). In general, microprojectiles are about one micron (10⁻⁶ m) in diameter, and are made of an inert material (gold or tungsten). The microprojectiles may be coated with DNA, and may be accelerated using gunpowder, an electric shock or gases under pressure (for example, compressed helium). In this manner, DNA may be introduced into practically any tissue of any vegetable species.

However, the process has a disadvantage: the lack of control over the incorporation of the gene into the plant genome. The transgene may break during the process and, therefore, fragments of the initial DNA may be integrated, or too many transgenes may be integrated and, therefore, the plant may react by silencing the transgene, thereby preventing the expression thereof.

The co-culturing of cells or tissues with *Agrobacterium tumefaciens* is the most widely used process to transform dicotyledonous plants. Until very recently, it was not possible to use it in monocotyledonous plants, a group that includes gramineal plants, which are very important for human nutrition, but it has already been achieved with rice and corn.

Bacteria of the genus *Agrobacterium* are plant pathogens capable of inducing a malformation called *gall tumour.* These bacteria penetrate into intercellular spaces through small wounds present in the plant, attracted by substances that the plant excretes on its open areas. Formation of the tumour takes places by the transfer of a DNA segment, the T-DNA present in an *Agrobacterium* plasmid, into the infected cell nuclei. In this way, the bacteria establish a kind of "genetic colonisation" of the plant, forcing it to manufacture a substance that only the *Agrobacterium* may use as a nutrient and which is segregated in the tumour.

The existence of virulence genes and tumour-inducing genes was observed. The latter are flanked by characteristic nucleotide sequences on the left and right borders. By means of genetic manipulation, *Agrobacterium* strains without tumour genes were obtained that maintained the left and right borders. In this way, any gene integrated within these borders will be transferred to the plant cells.

Once the transgene is introduced into the *Agrobacterium,* it is necessary to co-culture the plant cells with the bacteria. To this end, vegetable tissues are used which must be wounded in order to activate the bacterial virulence genes and thus induce introduction of the transgene. The vegetable tissues used may be from the leaves, cotyledons, stem or tubercle fragments, or even germinating seeds.

This system is more reliable than others, since the transformation is more stable and, in general, only one copy of the transgene is introduced.

All the transformation systems developed thus far require selecting the plants that contain the transgene introduced and eliminating the rest. The simplest system consists of incorporating an antibiotic- or herbicide-resistant gene into the transgene, in order to ensure, when preparing the *in vitro* culture in the presence of the selection agent (antibiotic or herbicide), that only those that have been transformed survive.

As previously described, the FT gene and the genes homologous to the FT gene of *Arabidopsis thaliana* are responsible for tuberization in plants. Therefore, modulation of the expression of these genes may be of interest to willfully induce tuberization in those plants that integrate them; i.e. not only induce, but also delay tuberization, for example, in the event that the plant does not have the adequate size to obtain an optimal yield. Thus, regulation at certain temperatures of the expression of the FT gene, or the homologues thereof, by means of promoters which induce expression of the gene that they regulate would make it possible, for example, to regulate the tuberization process in a nursery, by controlling the temperature conditions.

The expression "that modulates the activity", as used herein, primarily refers to inhibiting (decreasing) or increasing the level of activity of the FT protein, or the proteins orthologous or homologous to FT. The activity of FT (or of the homologous proteins thereof) may be modulated by modifying the levels and/or the activity of FT, or modifying the levels whereat the FT gene is transcribed, such that the level of activity of the FT proteins is modulated.

The invention is based on the fact that the FT gene and the homologues thereof are involved in the tuberization process. Therefore, in addition to the transgenic approach described, the invention may also be performed in non-transgenic plants, for example, in a *Solanaceae* plant, in particular of the genus Solanum and, more particularly, of the species Solanum tuberosum, by incorporating a gene homologous to the gene that encodes the Flowering Locus T (FT) protein into the genome thereof, where the gene homologous to FT is capable of being expressed at temperatures greater than 17ºC.

Therefore, modifications in the *FT* genes or any of the homologues thereof, including the SP6A orthologous gene, would make it possible to modulate the tuberization of a plant. Since this invention comprises the sequence of this gene, as well as the protein whereto it is translated, obtaining plants with modified tuberization may be performed by several methods.
- By the selection of spontaneous mutants: it must be taken into consideration that, in every cell division, there is a small probability of a genetic change occurring; it is not surprising, therefore, that, in a large cell mass, the population is heterogeneous. This distribution may cause yield problems, since, in general, variants have lower production levels than the parental population. These definitive changes (mutations) must be distinguished from phenotypic variations, which are dependent on the environmental conditions and take place in all the population cells that express the same physiological modification, within the variations allowed by their genotype. In spontaneous mutations, if the element responsible for the mutation is not known, it is very difficult to distinguish these phenotypic variations from those that present modifications in the genes responsible for tuberization, which are stable and hereditary. The present invention provides the necessary tools to select those mutants not only by observing the morphological characteristics of interest, but also detecting mutations in the genes responsible for tuberization (*FT* or any of the homologues thereof), by means of a simple selective screening process for a particular type of mutants. For example, it would be possible to morphologically select those plants, preferably of the family *Solanaceae,* that are capable of tuberising at temperatures greater than 17ºC, preferably at temperatures greater than 20ºC or 25ºC, more preferably, greater than 30ºC or 35ºC, and, even more preferably, at temperatures greater than 37ºC, and subsequently verify whether the *FT* genes or any of the homologues thereof that the plant presents in its genome show mutations with respect to a control wild genotype.

The term "genotype", as used in this specification, refers to the hereditary or genetic constitution of an individual; all the genetic material contained in a cell, which, in general, is called nuclear material.

The term "phenotype", as used in this specification, refers to the total sum of observable structural and functional properties of an organism that are the result of interaction between the genotype and the environment.

The term "type" refers to the plant designated as the type of a genus, subgenus, species or other taxonomic category; from a taxonomic standpoint, the "type" is the simple taxon element whereto the name is permanently assigned and whereon all the descriptive characteristics that meet the valid availability or publication conditions are based. This specification also discloses a control plant whereto other plants of the same taxonomic category are compared in order to determine whether they present modified tuberization, in order to subsequently analyse whether the gene homologous to *FT* shows mutations with respect to the genes of the control plant. This makes it possible to distinguish those tuberization modifications that are caused by physiological, environmental or other types of factors from those caused by mutations in the gene homologous to *FT*.

The induced mutation process involves two steps: treatment of the population with the selected mutagen and, thereafter, isolation of the mutants for their subsequent assay and selection. Inducing mutations in a plant is a very valuable tool for the improvement of plants, especially when it is desired to improve one or two easily identifiable characteristics in a well-adapted species or variety. Moreover, it has the advantage that the variability caused by the induced mutations is not essentially different from that caused by spontaneous mutations during evolution. In general, the choice of a mutagenic agent is dependent on practical considerations. In some cases, it is more convenient to use more than one mutagenic agent, instead of the massive use of a single one. As far as possible, isolation of the mutant should use the improved characteristic thereof (the vegetable architecture of interest) as the selection factor. Mutagenic agents may be grouped into physical agents (ultraviolet light, X-rays, gamma radiation, beta radiation, fast neutrons, heavy-ion beams, etc.) and chemical agents. Most chemical mutagens belong to the group of alkylating agents (ethyl methanesulfonate (EMS), diethyl sulfate (dES), etc.), but there are other groups, such as base analogues (such as 5-acyl bromide and 2-aminopurine) and structural mutagens (such as proflavine or acridine orange).

Mutagens primarily create point mutations and small deletions, insertions, transversions and/or transitions (of about 1 to 5 nucleotides). For example, without being limited thereto, they could be mutagens such as methyl methanesulfonate (MMS), Nitrosoguanidine (NTG), N-ethyl-N-nitrosourea (ENU), triethylmelamine (TEM), N-methyl-N-nitrosourea (MNU), procarbazine, chlorambucil, cyclophosphamide, diethyl sulfate, acrylamide monomer, melphalan, vincristine, dimethylnitrosamine, N-methyl-N'-nitro-Nitrosoguanidine (MNNG), nitrosoguanidine, 2-aminopurine, 7,12-dimethyl-benz(a)anthracene (DMBA), ethylene oxide, hexamethylphosphoramide, bisulfan, diepoxyalkanes (diepoxyoctane (DEO), diepoxybutane (BEB)), 2-methoxy-6-chloro-9[3-(ethyl-2-chloroethyl)aminopropylamine], acridine dihydrochloride (ICR-170), formaldehyde, etc.

Thus, for example, without being limited thereto, the seeds are subjected to the action of chemical mutagens, which leads to a number of mutations in the genome of said seeds. Said seeds are grown, leading to adult plants (M1), which self-pollinate, leading to an M2 generation. The DNA of the M2 plants is subjected to screening in order to determine whether it presents mutations in the gene of interest. Once the mutation is identified in the gene of interest, the seeds of the M2 plants that carry said mutation are grown, leading to M3 plants, which are subjected to screening in order to determine whether they exhibit the phenotypic characteristics associated with the gene of interest.

Thus, another aspect of the invention relates to a method of obtaining plants capable of tuberising at temperatures different from those whereat a control type plant of the same taxonomic category tuberises, which comprises:
a) obtaining vegetable material from a plant (parental),
b) subjecting the vegetable material of step (a) to a mutagenesis process,
c) culturing the mutated vegetable material until a complete plant is regenerated, and the offspring thereof,
d) analysing the offspring of the plants of step (c) in order to detect at least one mutation in the gene homologous to *FT*, or in the promoter thereof, and
e) selecting the offspring with at least one mutation in the gene homologous to *FT*, or in the promoter thereof, capable of tuberising under different environmental conditions, such as, for example, without being limited thereto, at temperatures different from those whereat a control type plant tuberises.

A preferred embodiment of this aspect of the invention optionally comprises cultivating the plant selected in step (e) in order to obtain offspring that present said capacity to tuberise under different environmental conditions, such as, for example, without being limited thereto, at temperatures different from those whereat a control type plant tuberises. Preferably, the plant may be taxonomically classified as belonging to the genus *Solanum* and, more preferably, to the species *Solanum tuberosum* L. In another preferred embodiment, the plant is capable of tuberising at temperatures greater than 17ºC, more preferably, at temperatures greater than 20ºC, 25ºC, 30ºC, 35ºC, or, more preferably, at temperatures greater than 37ºC.

A person skilled in the art will understand that a variety of vegetable material may be subjected to the mutagenesis process, including, without being limited thereto, plant seeds, pollen, cells or tissues. The type of vegetable material that is subjected to mutagenesis modifies the step wherein the plant DNA is subjected to screening in order to find the mutation. Thus, for example, when pollen is subjected to mutagenesis prior to the pollination of a non-mutagenic plant, the resulting seeds lead to M1 plants. Each cell of said M1 plants may contain the mutations induced in the pollen; for this reason, it is not necessary to wait for the M2 generation in order to perform the screening. This process is known as *tilling* in the state of the art.

The gene homologous to FT comprises a nucleotide sequence that encodes the amino acid sequence comprised in SEQ ID NO: 1 or an amino acid sequence that is at least 85%, preferably at least 90%, more preferably at least 95%, and even more preferably at least 99% identical to the amino acid sequence of SEQ ID NO: 1. In a preferred embodiment, the gene orthologous to FT comprises nucleotide sequence SEQ ID NO: 1, or any variant thereof.

It is possible to identify a plant that integrates said gene when it carries molecular markers related to the gene homologous to FT in its genome.

According to another aspect, the gene homologous to FT is under the regulation of a promoter that is induced in response to temperatures greater than 17ºC, in particular temperatures greater than 20ºC, preferably greater than 25ºC, more preferably greater than 30ºC, even more preferably temperatures greater than 35ºC, and even more preferably greater than 37ºC. In a preferred embodiment, the promoter is a heat-shock promoter and, in an even more preferred embodiment, it is the soybean (*Glycine max*) heat-shock promoter (HSP).

In a first embodiment of this aspect, the invention relates to a plant of the family *Solanaceae,* which is a transgenic plant of the species *Solanum tuberosum* that comprises the SP6A gene of *Solanum tuberosum* ssp. *andigena* in its genome under the regulation of the soybean (*Glycine max*) heat-shock promoter (HSP). Said plant of the family *Solanaceae* may be obtained, for example, by the transformation of a host *Solanaceae* plant with a genetic construct that comprises a gene homologous to FT, as defined above.

Alternatively, however, the plants of the family *Solanaceae* may be obtained by mutating wild *Solanaceae* plants and selecting the plant that shows tuberization at temperatures greater than 17ºC, or by the introgression of a gene homologous to FT from another, related wild plant that is capable of tuberising at high temperatures.

Furthermore, the invention relates to the offspring of the plants of the family *Solanaceae,* obtained by transgenesis or by mutation or introgression of other related wild plants. Another aspect of the invention relates to parts of said plants, which are selected from the group formed by cells, ovules, pollen, embryos, cotyledons, hypocotyls, roots, root apices, anthers, flowers, seeds, stems, tubercles, microtubercles, minitubercles, protoplasts, or a culture of regenerateable tissues or cells of such plants or produced from a tissue selected from the group formed by leaves, pollen, embryos, cotyledons, hypocotyls, meristematic cells, roots, root apices, anthers, flowers, seeds, stems, tubercles. Moreover, this invention relates to plants of the family *Solanaceae* regenerated from the cell cultures described above, where the regenerated plant has all the morphological and physiological characteristics of the plant of the invention.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following examples and drawings are provided for illustrative purposes, and are not intended to limit the scope of the present invention.

### DESCRIPTION OF THE FIGURES

Figure 1.- Diagram of the construct used to generate the transgenic lines.
Figure 2.- Levels of expression of the *SP6A* messenger in the different transgenic lines of potato. (-) RNA from leaves of untreated plants. (+) RNA from leaves of plants incubated for 30 min at 37ºC, in order to induce the expression of the HSP promoter. 20 µg of RNA were loaded per lane.
Figure 3.- HSP induction temperature. Replicas of transgenic line 1 were incubated at 30ºC, 35ºC, 37ºC, 40ºC and 45ºC, for 30 min. The samples labelled 22ºC were from the greenhouse and were not subjected to any treatment. 30 µg of RNA were loaded per lane.

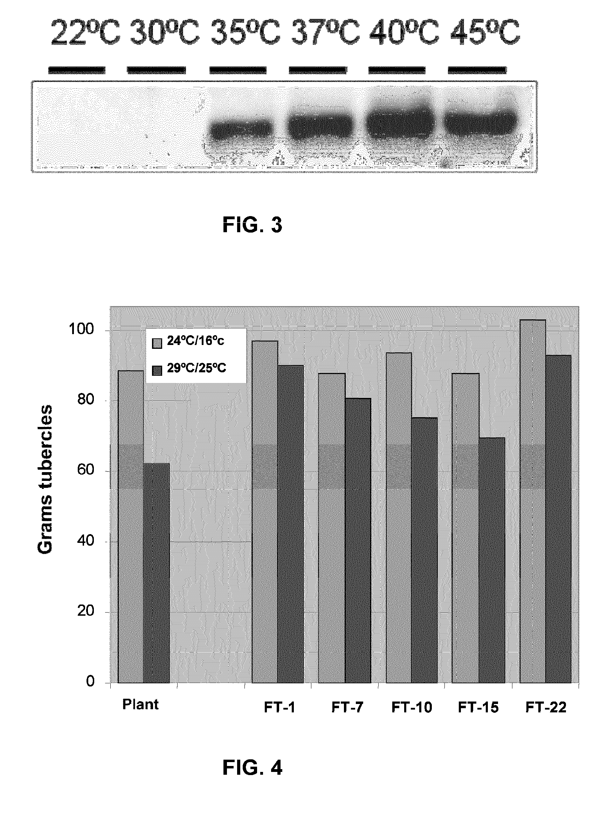

Figure 4.- Yield of tubercles of the transgenic lines under heat stress conditions. The different transgenic lines and plants of the Spunta variety were grown in the greenhouse until they reached a size of 10 leaves and transferred to 2 controlled-temperature chambers, where they were subjected to control conditions (24ºC day and 16ºC night) and heat stress conditions (29ºC day and 25ºC night). The yield of tubercles was determined after 1 month of cultivation under these conditions. Furthermore, the plants subjected to heat stress were treated at 37ºC for 30 min in the middle of the day, in order to induce expression of the transgene. The transgenic lines show a lower reduction in the yield of tubercles under heat stress conditions than the Spunta control.

### EXAMPLES

Below we will illustrate the invention by means of assays performed by the inventors.

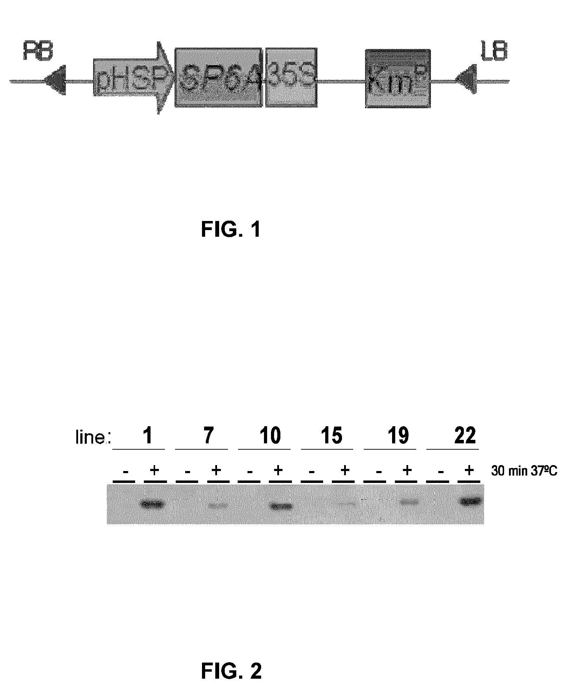

The HSP:SP6A construct was generated by amplification of the soybean *HSP* promoter with the HSP-for (SEQ ID NO: 4) and HSP-rev (SEQ ID NO: 5) primers, which generate the *Hind*III and Spel restriction sites at both ends. The PCR product was cut with these enzymes and cloned in the *Hind*/II/*Spe*I targets of the pK7WGF2 vector in order to substitute the 35S promoter with HSP. In order to introduce the GATEWAY recombination sequences without GFP fusion, the LucTrap-3 (GW) vector was used, which was digested with *Nco*I and *Xba*I in order to eliminate luciferase fusion. The cohesive ends generated were filled with Klenow and ligated, to generate the LucTrap-3ΔLUC vector. Said vector was digested with *Stu*l*lEco*Rl and the fragment generated was filled with Klenow and cloned in the *Spe*I/*Xba*I targets of HSPpK7WGF2, also filled with Klenow, to generate the HSP(GW) vector. The coding region corresponding to the SP6A gene was amplified with the SP6ATOPOfor (SEQ ID NO: 6) and SP6Arev (SEQ ID NO: 7) primers. The PCR product was inserted into the pENTR^{™}/D-TOPO® vector, and cloned in the HSP(GW) vector by incubation with LR clonase. The construct thus generated (Fig. 1) was transformed into the pGB3101 strain of *Agrobacterium tumefaciens* and used to infect potato leaves from the Spunta variety, following the method described by Banerjee et al., 2006. Plant Sci 170: 732-738.

The transformed shoots were selected in a regeneration medium containing 50 mg/l of Kanamycin and allowed to take root in the presence of the antibiotic. The transgenic plants thus generated were transferred to the greenhouse and, once they reached a size of 5 leaves, were treated for 30 min at 37ºC, in order to induce expression of the transgene.

RNA was isolated from the leaves of untreated plants and plants subjected to heat shock, and hybridised against a fragment corresponding to the SP6A gene (Fig. 2). Thus, lines 1, 7, 10, 15, 19 and 22, which show different transgene activation levels following the treatment at 37ºC, were selected. These lines were propagated *"in-vitro"* in order to obtain a minimum of 10 replicas per line, and transferred to the greenhouse jointly with control plants of the Spunta variety propagated in an identical manner. Once the plants had reached a size corresponding to 10 leaves, they were divided into 2 groups (5 replicas per group), which were transferred to controlled-temperature culture chambers. These were regulated at 24ºC during the day and 16ºC during the night (control conditions) or 29ºC during the day and 25ºC during the night (heat stress conditions). Furthemore, the plants subjected to heat stress were treated with a 37ºC heat shock for 30 min in the middle of the day, in order to ensure activation of the transgene.

Both groups of plants were cultivated for 30 days under these conditions; once this time had elapsed, the tubercles were collected and the weight, in tubercles per plant, was determined. As shown in Fig. 4, under control conditions, the transgenic lines showed a yield of tubercles that was similar or slightly greater than that of the Spunta control. Under heat stress conditions, Spunta showed a 30% reduction in the yield of tubercles (from 89 g of tubercles/plant under control conditions to 62 g/plant under heat stress conditions). This reduction was much smaller in the transgenic lines, where it ranged between 23% (from 91 g/plant to 70 g/plant under heat stress conditions) for line 15, with lower transgene expression levels, and 8% (from 97 g/plant to 89 g/plant) for line 1, with higher transgene expression levels. The number of tubercles was similar in the transgenic lines and the control, with an equivalent average size of the tubercles in both lines.

These results indicate that overexpression of the *SP6A* gene under the control of a heat-inducible promoter significantly improves the yield of tubercles under heat stress conditions. On the other hand, the use of a heat-inducible promoter makes it possible for the transgene not to be active if the temperatures do not exceed a certain threshold, and to activate only under temperature conditions that are known to exert an adverse effect on the formation and differentiation of tubercles.

In order to determine the minimum temperature threshold whereat the transgene is activated, replicas of line 1 were subjected to temperatures of 30ºC, 35ºC, 37ºC, 40ºC and 45ºC for 30 min. RNA was isolated from the leaves of these plants, and from plants grown in the greenhouse (22ºC), which were used as a negative control. These RNAs were hybridised with a probe corresponding to the SP6A transcript and, as shown in Fig. 3, it was observed that activation of the transgene begins at 35ºC and reaches a maximum at 40ºC, the gene remaining inactive at temperatures below 30ºC. This would indicate that, in plants grown in the field, these lines will behave like the control lines at temperatures below 30ºC, but there will be activation of the transgene and, consequently, of tuberization when the temperature exceeds this threshold, conditions which are known to have a strong negative impact on the yield of tubercles.

## Claims

1. Isolated RNA or DNA polynucleotide capable of being translated into an amino acid sequence which comprises a peptide that shows an identity with respect to SEQ ID NO: 1 selected from any of the following:
a. at least 85%,
b. at least 90%,
c. at least 95%, or
d. 99%.

2. Isolated RNA or DNA polynucleotide capable of being translated into amino acid sequence SEQ ID NO: 1.

3. DNA or RNA genetic construct which comprises one of the following types of sequences:
a. nucleotide sequence that comprises, at least, the polynucleotide according to claim 1 or the coding sequence of SEQ ID NO: 1, for the *in vitro* or *in vivo* transcription thereof, or
b. nucleotide sequence, corresponding to a gene expression system or vector, which comprises the polynucleotide according to any one of claims 1-2, operatively linked to, at least, one promoter that directs the transcription of said nucleotide sequence, and to other necessary or appropriate sequences for the adequate transcription and regulation in time and space thereof.

4. Genetic construct according to claim 3, wherein the promoter is capable of directing the transcription of a polynucleotide according to any one of claims 1-2 at temperatures greater than a temperature selected from any of the following:
a. 17ºC,
b. 20ºC,
c. 25ºC,
d. 30ºC,
e. 35ºC, or
f. 37ºC.

5. Genetic construct according to any one of claims 3-4, wherein the promoter is a heat-shock gene promoter.

6. Genetic construct according to any one of claims 3-5, wherein the promoter is the soybean heat-shock gene promoter (SEQ ID NO: 3).

7. Use of the genetic construct according to any one of claims 3-6, to express the amino acid sequence according to any one of claims 1-2.

8. Use of a polynucleotide homologous to the isolated FT (*FLOWERING LOCUS T*) gene, to modulate the tuberization process in a plant.

9. Use of a polynucleotide homologous to the isolated FT (*FLOWERING LOCUS T*) gene according to claim 8, wherein the polynucleotide homologous to the *FT* gene is a polynucleotide according to any one of claims 1-2.

10. Use of a polynucleotide homologous to the isolated FT (*FLOWERING LOCUS T*) gene according to the preceding claim, wherein the polynucleotide homologous to the isolated FT gene is linked to a heat-shock promoter or any other inducible promoter.

11. Use of a polynucleotide homologous to the isolated FT (*FLOWERING LOCUS T*) gene according to claim 10, wherein the promoter is the soybean heat-shock gene promoter (SEQ ID NO: 3).

12. Use of the genetic construct according to any one of claims 3-6, to modulate tuberization in a plant.

13. Use of the polynucleotide or a genetic construct according to any one of claims 8-12, wherein the plant belongs to the genus *Solanum* L.

14. Use of the polynucleotide or the genetic construct according to the preceding claim, where the plant belongs to the species *Solanum tuberosum* L.

15. Use of the polynucleotide or the genetic construct according to any one of claims 13-14, where the plant belongs to the *Spunta* variety of the species *Solanum tuberosum* L.

16. Seed the genetic material whereof integrates:
a. a polynucleotide according to any one of claims 1-2,
b. a genetic construct according to any one of claims 3-6, or
c. a polynucleotide homologous to the isolated *FT* (*FLOWERING LOCUS T*) gene.

17. Vegetable cells the genetic material whereof integrates:
a. a polynucleotide according to any one of claims 1-2,
b. a genetic construct according to any one of claims 3-6,
c. a polynucleotide homologous to the isolated *FT* (*FLOWERING LOCUS T*) gene.

18. Culture of vegetable cells according to claim 17.

19. Group of cells according to claim 17, which form the tubercles, minitubercles or microtubercles.

20. Plant that comprises cells according to claim 17.

21. Plant according to the preceding claim, which is obtained by the growth of a cell according to claim 17, a group of cells according to any one of claims 18-19, or the seed according to claim 16.

22. Plant that is capable of tuberising under environmental conditions different from those whereat a control type plant of the same taxonomic category tuberises, due to transgenic or non-transgenic mutations in the gene homologous to the *FT* gene, or the promoter thereof.

23. Pollen grain the genetic material whereof is a derivative of the genetic material of a cell according to any one of claims 17-19, or comes from a plant according to claim 22.

24. Seed, cell, cell culture, plant or pollen grain according to any one of claims 16-23, which can be taxonomically classified as belonging to the genus *Solanum.*

25. Seed, cell, cell culture, plant or pollen grain according to any one of claims 16-24, which can be taxonomically classified as belonging to the species *Solanum tuberosum* L.

26. Plant according to any one of claims 20-22, which is capable of tuberising at temperatures greater than a temperature selected from any of the following:
a. 17ºC,
b. 20ºC,
c. 25ºC,
d. 30ºC,
e. 35ºC, or
f. 37ºC.

27. Use of a polynucleotide homologous to the FT gene according to any one of claims 10-12, where the polynucleotide homologous to the *FT* gene, operatively linked or not to a heat-shock promoter, is integrated into the genetic material of the seed, cell, cell culture, plant or pollen grain according to any one of claims 16-26.

28. Method for obtaining plants capable of tuberising under environmental conditions different from those whereat a control type plant of the same taxonomic category tuberises, which comprises:
a. transfecting a polynucleotide according to any one of claims 1-2, the genetic construct according to any one of claims 3-6, or a polynucleotide homologous to the isolated FT (*FLOWERING LOCUS T*) gene, into a host cell or vegetable cell culture,
b. growing the host cell or vegetable cell culture in a suitable medium,
c. regenerating a complete plant.

29. Method for obtaining plants capable of tuberising under environmental conditions different from those whereat a control type plant of the same taxonomic category tuberises, which comprises:
a. obtaining vegetable material from a plant,
b. subjecting the vegetable material of step (a) to a mutagenesis process,
c. cultivating the mutated vegetable material until a complete plant is regenerated, and the offspring thereof,
d. analysing the offspring of the plants of step (c), in order to detect at least one mutation in the gene homologous to *FT,* or in the promoter region thereof, and
e. selecting the offspring that have at least one mutation in the gene orthologous to *FT,* or in the promoter region thereof, which present a modified tuberization process.

30. Method according to the preceding claim, which further comprises cultivating the selected plant in order to obtain offspring that present said modification in the control of the tuberization process.

31. Method according to any one of claims 29-30, where the mutagenesis process of step (b) is performed by means of irradiation or chemical mutagens.

32. Method according to any one of claims 28-31, where the plant may be taxonomically classified as belonging to the genus *Solanum.*

33. Method according to any one of claims 28-32, where the plant may be taxonomically classified as belonging to the species *Solanum tuberosum* L.

34. Method according to any one of claims 28-33, where the plant is capable of tuberising at temperatures greater than a temperature selected from any of the following:
a. 17ºC,
b. 20ºC,
c. 25ºC,
d. 30ºC,
e. 35ºC, or
f. 37ºC.


<110>  Consejo Superior de Investigaciones Científicas (CSIC)

<120>  GENES HOMOLOGOUS TO THE FLOWERING LOCUS T (FT) GENE AND THE USE
       THEREOF TO MODULATE TUBERIZATION

<130>  EP1641.142

<150>  ES200930114
<151>  2009-04-29

<150>  PCT/ES2010/070265
<151>  2010-04-26

<160>  9

<170>  PatentIn version 3.5

<210>  1
<211>  173
<212>  PRT
<213>  Solanum tuberosum

<400>  1

Met Pro Arg Val Asp Pro Leu Ile Val Gly Arg Val Ile Gly Glu Val
1               5                   10                  15

Leu Asp Pro Phe Thr Arg Ser Val Asp Leu Arg Val Val Tyr Asn Asn
            20                  25                  30

Arg Glu Val Asn Asn Ala Cys Val Leu Lys Pro Ser Gln Val Val Met
        35                  40                  45

Gln Pro Arg Val His Ile Gly Gly Asp Asp Leu Arg Asn Phe Tyr Thr
    50                  55                  60

Leu Ile Met Val Asp Pro Asp Ala Pro Ser Pro Ser Asp Pro Asn Leu
65                  70                  75                  80

Arg Glu Tyr Leu His Trp Leu Val Thr Asp Ile Pro Ala Thr Thr Asn
                85                  90                  95

Thr Ser Phe Gly Asn Glu Val Val Cys Tyr Glu Asn Pro Thr Pro Thr
            100                 105                 110

Met Gly Ile His Arg Phe Ile Leu Ala Leu Phe Arg Gln Ser Arg Arg
        115                 120                 125

Glu Thr Val Tyr Ala Pro Gly Trp Arg Gln Asn Phe Asn Thr Arg Asp
    130                 135                 140

Phe Ala Glu Leu Tyr Asn Leu Gly Leu Pro Val Ala Ala Val Tyr Phe
145                 150                 155                 160

Asn Cys His Arg Glu Ser Gly Thr Gly Gly Arg Arg Ala
                165                 170

<210>  2
<211>  175
<212>  PRT
<213>  Arabidopsis thaliana

<400>  2

Met Ser Ile Asn Ile Arg Asp Pro Leu Ile Val Ser Arg Val Val Gly
1               5                   10                  15

Asp Val Leu Asp Pro Phe Asn Arg Ser Ile Thr Leu Lys Val Thr Tyr
            20                  25                  30

Gly Gln Arg Glu Val Thr Asn Gly Leu Asp Leu Arg Pro Ser Gln Val
        35                  40                  45

Gln Asn Lys Pro Arg Val Glu Ile Gly Gly Glu Asp Leu Arg Asn Phe
    50                  55                  60

Tyr Thr Leu Val Met Val Asp Pro Asp Val Pro Ser Pro Ser Asn Pro
65                  70                  75                  80

His Leu Arg Glu Tyr Leu His Trp Leu Val Thr Asp Ile Pro Ala Thr
                85                  90                  95

Thr Gly Thr Thr Phe Gly Asn Glu Ile Val Cys Tyr Glu Asn Pro Ser
            100                 105                 110

Pro Thr Ala Gly Ile His Arg Val Val Phe Ile Leu Phe Arg Gln Leu
        115                 120                 125

Gly Arg Gln Thr Val Tyr Ala Pro Gly Trp Arg Gln Asn Phe Asn Thr
    130                 135                 140

Arg Glu Phe Ala Glu Ile Tyr Asn Leu Gly Leu Pro Val Ala Ala Val
145                 150                 155                 160

Phe Tyr Asn Cys Gln Arg Glu Ser Gly Cys Gly Gly Arg Arg Leu
                165                 170                 175

<210>  3
<211>  437
<212>  DNA
<213>  Glycine max

<400>  3
aagcttctcg agagtcagcc ttttaagaga tagaatttaa aatataattt gcgtaaaaca       60

ttattaaaaa tacaaattta taaattaagt tcaactcatc ctatctcact ctttaaatac      120

gatgtttact tattagactc attaataaaa aaaaaaaaaa tcatttgtac aaagcccacc 180

ataaaggcaa tttgggcctg gtagaccaat cctaaccaat gtctggttaa gatggtccaa 240

tcccgaaact tctagttgcg gttcgaagaa gtccagaatg tttctgaaag tttcagaaaa 300

ttctagtttt gagattttca gaagtacggc atgatgatgc ataacaagga ctttctcgaa 360

agtactatat tgctcctcta catcatttta aataccccat gtgtcctttg aagacacatc 420

acagaaagaa gactagt 437

<210> 4
<211> 16
<212> DNA
<213> Artificial

<220>
<223> cebador HSP-for

<400> 4
gcaagcttct cgagag 16

<210> 5
<211> 26
<212> DNA
<213> Artificial

<220>
<223> cebador HSP-rev

<400> 5
gactagtctt ctttctgtga tgtgtc 26

<210> 6
<211> 25
<212> DNA
<213> Artificial

<220>
<223> cebador SP6ATOPOfor

<400> 6
caccatgcct agagttgatc cattg 25

<210> 7
<211> 20
<212> DNA
<213> Artificial

<220>
<223> cebador SP6Arev

<400> 7
ttatgcgcga cgtcctccag 20

<210> 8
<211> 522
<212> DNA

<213>  Solanum tuberosum

<400>  8
atgcctagag ttgatccatt gatagttggt cgtgtgatag gggatgtttt agatccattc      60

actaggtctg ttgatcttag agttgtttat aataataaag atgtgaacaa tgcatgtgtg     120

ttgaaacctt cacaagttgt tatgcaacct agggttcata ttggagggga cgatcttcgc     180

aacttttaca ctctgattat ggtggatcct gatgctccaa gcccaagcga ccctaacttg     240

agggagtatc tacattggct ggtcacagat atcccagcaa ctacaaatac aagctttgga     300

aatgaagtcg tatgctacga gaatccaaca cctacgatgg gaattcatcg attcattttg     360

gctttatttc gacaatcgag acgtgaaaca gtgtatgccc caggttggcg tcaaaatttc     420

aacacaagag actttgctga gctttacaat cttggattgc ctgttgcagc tgtttacttc     480

aattgccata gggagagtgg tactggagga cgtcgcgcat aa                        522


<210>  9
<211>  528
<212>  DNA
<213>  Arabidopsis thaliana

<400>  9
atgtctataa atataagaga ccctcttata gtaagcagag ttgttggaga cgttcttgat      60

ccgtttaata gatcaatcac tctaaaggtt acttatggcc aaagagaggt gactaatggc     120

ttggatctaa ggccttctca ggttcaaaac aagccaagag ttgagattgg tggagaagac     180

ctcaggaact tctatacttt ggttatggtg gatccagatg ttccaagtcc tagcaaccct     240

cacctccgag aatatctcca ttggttggtg actgatatcc ctgctacaac tggaacaacc     300

tttggcaatg agattgtgtg ttacgaaaat ccaagtccca ctgcaggaat tcatcgtgtc     360

gtgtttatat tgtttcgaca gcttggcagg caaacagtgt atgcaccagg gtggcgccag     420

aacttcaaca ctcgcgagtt tgctgagatc tacaatctcg gccttcccgt ggccgcagtt     480

ttctacaatt gtcagaggga gagtggctgc ggaggaagaa gactttag                  528